(19) Europäisches Patentamt
European Patent Office
Office européen des brevets

(11) Publication number: **0 083 565 A1**

# (12) EUROPEAN PATENT APPLICATION

(21) Application number: 83101609.2

(22) Date of filing: 01.04.81

(51) Int. Cl.³: C 07 D 413/04

(30) Priority: 10.04.80 US 138872
10.04.80 US 138873

(43) Date of publication of application: 13.07.83
Bulletin 83/28

(84) Designated Contracting States: AT BE CH DE FR GB IT LI LU NL SE

(60) Publication number of the earlier application in accordance with Art. 76 EPC: 0038298

(71) Applicant: SANDOZ AG, Lichtstrasse 35, CH-4002 Basel (CH)

(72) Inventor: Brand, Leonard Jay, 114 Dover Chester Road, Randolph, N.J. 07801 (US)
Inventor: Nadelson, Jeffrey, 12 Benedict Crescent, Denville, N.J. 07834 (US)

## (54) Isoxazolyl indolamines.

(57) A compound of formula XI

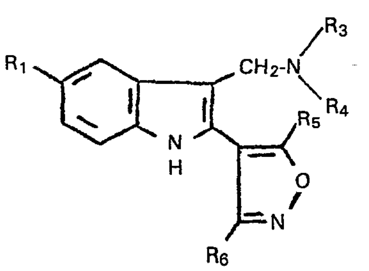

XI

wherein
$R_1$ represents hydrogen, fluorine, chlorine, $C_{1-4}$alkyl or $C_{1-4}$alkoxy,
$R_3$ and $R_4$ represent, independently, $C_{1-4}$alkyl or together with the adjacent nitrogen atom

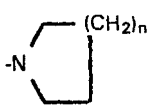 , 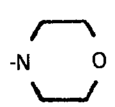 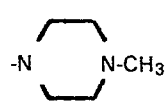

wherein n is 1, 2 or 3,
$R_5$ represents hydrogen or $C_{1-4}$alkyl and
$R_6$ represents hydrogen, $C_{1-4}$alkyl, phenyl or phenyl mono-substituted by fluorine, chlorine, $C_{1-4}$alkyl or $C_{1-4}$alkoxy.

These compounds may be used in the preparation of pharmacologically active substances.

EP 0 083 565 A1

0083565

Case 600-6887/III

ISOXAZOLYL INDOLAMINES

The invention relates to a compound of formula XI

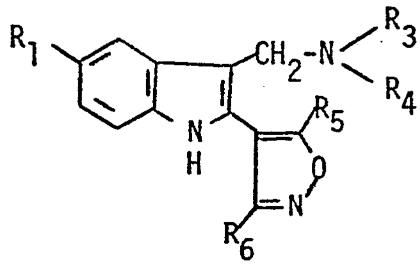

XI

wherein $R_1$ represents hydrogen, fluorine, chlorine, $C_{1-4}$alkyl or $C_{1-4}$alkoxy,
$R_3$ and $R_4$ represent, independently, $C_{1-4}$alkyl or together with the adjacent nitrogen atom

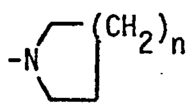 , 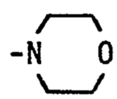 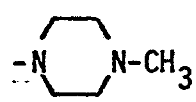

wherein n is 1, 2 or 3,
$R_5$ represents hydrogen or $C_{1-4}$alkyl
and $R_6$ represents hydrogen, $C_{1-4}$alkyl, phenyl or phenyl monosubstituted by fluorine, chlorine, $C_{1-4}$alkyl or $C_{1-4}$-alkoxy.

Compounds of formula XI can be prepared by reacting a compound of formula V

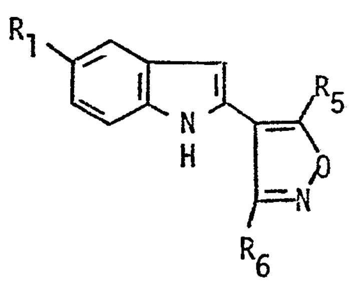

V

with a compound of formula IV

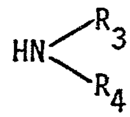

IV

whereby $R_1$, $R_3$, $R_4$, $R_5$ and $R_6$ are as defined above, in the presence of formaldehyde, an acid catalyst such as acetic acid and an organic co-solvent. Examples of suitable solvents are ethers such as diethylether, tetrahydrofuran and particularly dioxane. Preferred temperatures are from about -10° to +30°C, most preferably 0° to 10°C.

Preparation of compounds V is described in our co-pending application no.

The products resulting from the above processes can be isolated and purified in conventional manner. Intermediate compounds can where appropriate be further reacted without isolation..

Insofar as the production of other starting materials is not described above, these are either known or can be prepared in conventional manner from known starting materials.

The compounds of formula XI may be employed in the production of pharmacologically active end-products.

They may for example be reacted with an alkali metal cyanide such as sodium or potassium cyanide to produce a mixture of a compound of formula Xa

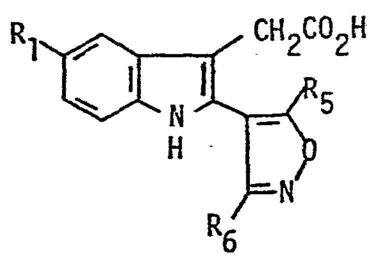

Xa

and formula Xb

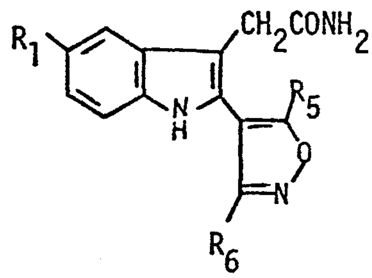

Xb

which, following conversion of Xb to Xa by conventional hydrolysis techniques, may in turn be converted into a compound of formula III

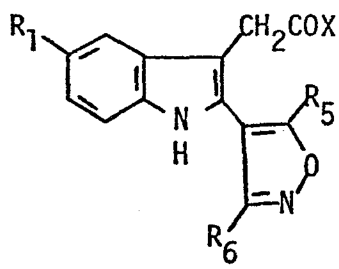

III

by introduction of the group X.

These compounds may then be reacted with a compound of formula IV

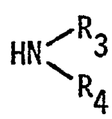

IV

to give a compound of formula II

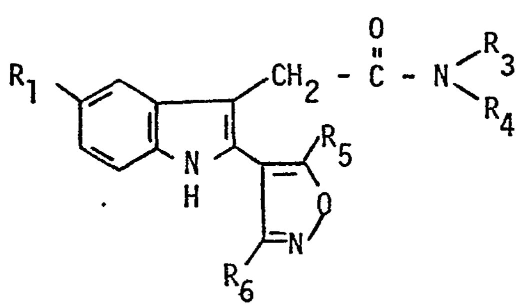

II

which in turn may be reduced to yield a pharmacologically active compound of formula I

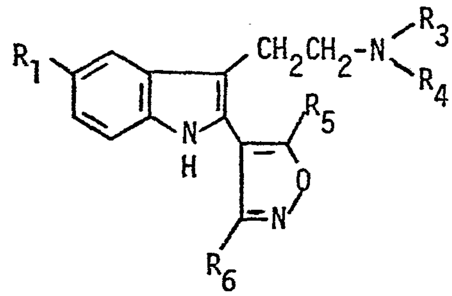

I

In the compounds of formulae I, II, III, IV, Xa and Xb, $R_1$, $R_3$, $R_4$, $R_5$ and $R_6$ are as defined for formula XI, and X represents a leaving group such as chlorine or bromine.

The end-products of formula I are described and claimed in our co-pending application no. 81810131.3 (Publ. No. 0038298/A1) which also describes the processes outlined above and preferred substituents.

The following Example in which all temperatures are in °C and room temperature is 20-30°C illustrate the invention.

EXAMPLE : 2-(3-Ethyl-5-methyl-4-isoxazolyl)-3-(dimethylamino-methyl)-indole (Compound of formula XI)

A mixture of 20.6 ml (0.24 mol) 37% aqueous formaldehyde, 18 ml (0.12 mol) 40% aqueous dimethylamine and 80 ml acetic acid is cooled to 0° and treated by the dropwise addition of 25.5 g (0.113 mol) 2-(3-ethyl-5-methyl-4-isoxazolyl)-indole in a solution of 45 ml acetic acid and 125 ml dioxane. After addition is complete the mixture is stirred for 1 hour at room temperature and poured onto 500 ml ice-water. The resulting solution is made basic with 20% potassium hydroxide and then extracted with methylene chloride. The methylene chloride is washed with water, brine, dried over anhydrous magnesium sulphate, filtered and evaporated to give a gum that crystallizes to give 2-(3-ethyl-5-methyl-4-isoxazolyl)-3-(dimethylaminomethyl)-indole; m.p. 88° to 90°

<u>What is claimed is:</u>

1. A compound of formula XI

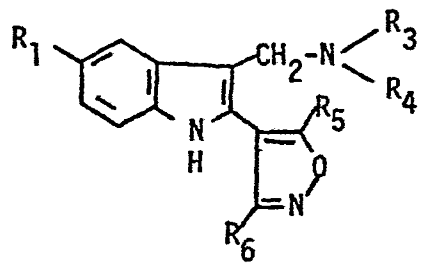

XI

wherein $R_1$ represents hydrogen, fluorine, chlorine, $C_{1-4}$alkyl or $C_{1-4}$alkoxy,
$R_3$ and $R_4$ represent, independently, $C_{1-4}$alkyl or together with the adjacent nitrogen atom

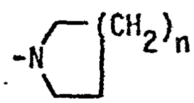 , 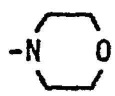 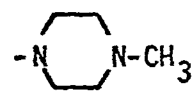

wherein n is 1, 2 or 3,
$R_5$ represents hydrogen or $C_{1-4}$alkyl
and $R_6$ represents hydrogen, $C_{1-4}$alkyl, phenyl or phenyl mono-substituted by fluorine, chlorine, $C_{1-4}$alkyl or $C_{1-4}$-alkoxy.

2. 2-(3-Ethyl-5-methyl-4-isoxazolyl)-3-(dimethylaminomethyl)-indole.

3. A process for preparing a compound of formula XI which comprises reacting a compound of formula V

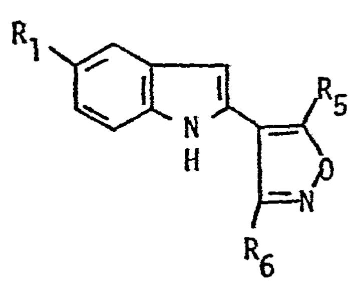

V

with a compound of formula IV

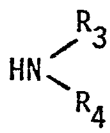

IV

whereby $R_1$, $R_3$, $R_4$, $R_5$ and $R_6$ are as defined above in the presence of formaldehyde, an acid catalyst such as acetic acid and an organic co-solvent.

0083565

European Patent Office

# EUROPEAN SEARCH REPORT

Application number

EP 83 10 1609

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | US-A-3 467 670 (JOHN T. SUH)<br>--- | | C 07 D 413/04 |
| A | US-A-4 059 583 (DAVID FRED McCOMSEY)<br>--- | | |
| A | GB-A-1 075 156 (ISTITUTO LUSO FARMACO D'ITALIA)<br>----- | | |
| | | | TECHNICAL FIELDS SEARCHED (Int. Cl. 3) |
| | | | C 07 D 413/00 |

The present search report has been drawn up for all claims

| Place of search | Date of completion of the search | Examiner |
|---|---|---|
| THE HAGUE | 14-04-1983 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82